# EUROPÄISCHE PATENTSCHRIFT

(11) **EP 3 096 674 B1**
(45) Veröffentlichungstag und Bekanntmachung des Hinweises auf die Patenterteilung: **13.03.2019**
(21) Anmeldenummer: 15701749.2
(22) Anmeldetag: 22.01.2015
(51) Int. Cl.: A61B 1/005, A61B 1/015, A61B 1/12, A61B 1/00

(54) **FLUIDBLOCK FÜR EIN ENDOSKOPBEDIENTEIL UND ENDOSKOP**
FLUID BLOCK FOR AN ENDOSCOPE CONTROL PART AND ENDOSCOPE
BLOC FLUIDIQUE POUR UN ORGANE DE COMMANDE D'UN ENDOSCOPE, ENDOSCOPE

(30) Priorität: 23.01.2014 DE 102014201208
(43) Veröffentlichungstag der Anmeldung: 30.11.2016
(73) Patentinhaber: Digital Endoscopy GmbH, 86316 Friedberg (DE)
(72) Erfinder: VIEBACH, Thomas, 86579 Waidhofen (DE); PAUKER, Fritz, 86420 Diedorf (DE)
(74) Vertreter: TBK
(86) Internationale Anmeldenummer: PCT/EP2015/051252
(87) Internationale Veröffentlichungsnummer: WO 2015/110528

(56) Entgegenhaltungen:
- EP-A1- 0 055 394
- JP-A- 2007 313 047
- US-A1- 2006 135 851
- US-B1- 6 383 132

## Beschreibung

Die vorliegende Erfindung bezieht sich auf einen Fluidblock für ein Endoskopbedienteil und auf ein Endoskop mit einem solchen Fluidblock.

In einem Endoskopbedienteil eines Endoskops sind üblicherweise z.B. ein Gasführungsschlauch zum Einleiten von Gas wie z.B. Aufblasgas und ein Flüssigkeitsführungsschlauch zum Einleiten einer Flüssigkeit wie z.B. einer Spülflüssigkeit vorgesehen. Darüber hinaus sind im Endoskopbedienteil Ventile für diese Kanäle vorgesehen, durch die der Anwender die Zufuhr von Gas/Flüssigkeit steuert. Diese Ventile sind mitunter sehr kompliziert und kostspielig.

Dadurch ergeben sich im Endoskopbedienteil viele Flächen, deren Reinigung schwierig sein kann, womit sich ein Risiko einer bleibenden Kontamination ergibt. Darüber hinaus ist der Zusammenbau und die Wartung eines solchen Endoskopbedienteil nicht einfach.

Die US 2006/135851 A1 offenbart einen Fluidblock für ein Endoskopbedienteil mit den Merkmalen des Oberbegriffs von Anspruch 1. Dieser Fluidblock hat einen einteiligen Aufbau.

Die US 6 383 132 B1 offenbart einen weiteren einteiligen Fluidblock für ein Endoskopbedienteil.

### DURCH DIE ERFINDUNG ZU LÖSENDE AUFGABE

Aufgabe der vorliegenden Erfindung ist es somit, eine verbesserte Möglichkeit zum Anordnen von Elementen in einem Endoskopbedienteil zu schaffen. Außerdem soll ein verbessertes Endoskop geschaffen werden.

### LÖSUNG DER AUFGABE

In Hinblick auf die verbesserte Möglichkeit zum Anordnen von Elementen eines Endoskopbedienteils ist diese Aufgabe erfindungsgemäß durch einen Fluidblock mit den Merkmalen von Anspruch 1 gelöst.

Ein Endoskop ist in den Ansprüchen 13 und 14 aufgezeigt.

Vorteilhafte Weiterbildungen sind Gegenstand der abhängigen Ansprüche.

Die Erfindung betrifft somit einen Fluidblock für ein Endoskopbedienteil, mit zumindest einem im Inneren des Fluidblocks vorgesehenen Gasführungskanal mit einer Eingangsöffnung und einer Ausgangsöffnung, zumindest einem im Inneren des Fluidblocks vorgesehenen Flüssigkeitsführungskanal mit einer Eingangsöffnung und einer Ausgangsöffnung, und zumindest einem Steuerventil zum Öffnen/Blockieren des zumindest einen Gasführungskanals und des zumindest einen Flüssigkeitsführungskanals.

In einem solchen Fluidblock kann die gesamte Fluidschaltung für das Endoskop untergebracht werden. Es sind lediglich für jeden Kanal ein Verbindungsstück für den Katheter und ein Verbindungsstück, das zum Endoskopstecker hin weist, erforderlich. Der Zusammenbau und die Wartung eines Endoskopbedienteils mit einem solchen Fluidblock ist einfach und wartungsfreundlich.

Im Fluidblock kann das Steuerventil so im Fluidblock angeordnet sein, dass es von der Außenseite des Fluidblocks betätigbar ist.

Im Fluidblock kann der Fluidblock aus transparentem Kunststoff hergestellt sein. Ein solcher Fluidblock kann kostengünstig hergestellt werden.

Im Fluidblock kann das Steuerventil in dem zumindest einen Gasführungskanal und in dem zumindest einen Flüssigkeitsführungskanal angeordnet sein, wobei das Steuerventil zwischen einer Gaszuführstellung, in der das Steuerventil den Gasführungskanal öffnet, und einer Flüssigkeitszuführstellung, in der das Steuerventil den Flüssigkeitsführungskanal öffnet, schaltbar ist.

Im Fluidblock kann das Steuerventil ein in zwei Schaltstellungen verschiebbares Schieberventil sein, wobei in der Gaszuführstellung das Steuerventil den Gasführungskanal öffnet und den Flüssigkeitsführungskanal schließt, und in der Flüssigkeitszuführstellung das Steuerventil den Flüssigkeitsführungskanal öffnet und den Gasführungskanal schließt; wobei das Steuerventil einen sich im Körper des Steuerventils erstreckenden Luftabgabekanal aufweist, dessen offenes Außenende außerhalb des Fluidblocks nach außen weist, und dessen Innenende in der Gaszuführstellung des Steuerventils mit dem Gasführungskanal in Kommunikation steht.

Im Inneren des Fluidblocks kann ein Arbeitskanal mit einer Eingangsöffnung und einer Ausgangsöffnung vorgesehen sein.

Im Fluidblock kann benachbart zum Steuerventil ein Arbeitskanalventil so im Fluidblock angeordnet sein, dass es von der Außenseite des Fluidblocks betätigbar ist, wobei das Arbeitskanalventil zwischen einer Stellung, in der der Arbeitskanal geöffnet ist, und einer Stellung, in der der Arbeitskanal geschlossen ist, schaltbar ist.

Im Fluidblock kann das Steuerventil und/oder das Arbeitskanalventil ein Wegwerfventil zur Einmalverwendung sein.

Der Fluidblock kann mit einem Kanal für zumindest eine elektrische Leitung für ein elektronisches Instrument am distalen Endoskopschlauchende, und Kanälen für Zugseile zur Deflectingsteuerung versehen sein.

Die Merkmale der Erfindung können geeignet kombiniert werden.

Nachstehend ist die Erfindung detailliert anhand von Beispielen erläutert.

### Kurzbeschreibung der Zeichnungen

Figur 1 zeigt ein Anwendungsbeispiel eines erfindungsgemäßen Fluidblocks.
Figur 2 zeigt eine perspektivische Explosionsansicht eines Endoskopbedienteils mit dem Fluidblock von Fig. 1.
Figur 3 zeigt eine perspektivische Explosionsansicht des Endoskopbedienteils mit dem Fluidblock von Fig. 2 unter Betrachtung von der Rückseite.
Figur 4 zeigt eine schematische Schnittansicht eines Steuerventils zum Öffnen/Blockieren eines Gasführungskanals und eines Flüssigkeitsführungskanals, wobei Fig. 4A eine Luftöffnungsstellung zeigt, Fig. 4B eine Luftzuführstellung zeigt und Fig. 4C eine Spülstellung zeigt.
Figur 5 zeigt eine schematische Schnittansicht eines Arbeitskanalventils, wobei
Fig. 5A eine Stellung, in der der Arbeitskanal geschlossen ist, zeigt, und Fig. 5B eine Stellung, in der der Arbeitskanal geöffnet ist, zeigt.
Figur 6 zeigt ein Anwendungsbeispiel eines erfindungsgemäßen Fluidblocks mit dem Steuerventil und dem Arbeitskanalventil der Figuren 4 und 5.

Nachstehend sind Ausführungsbeispiele der vorliegenden Erfindung beschrieben.

### Erstes Ausführungsbeispiel

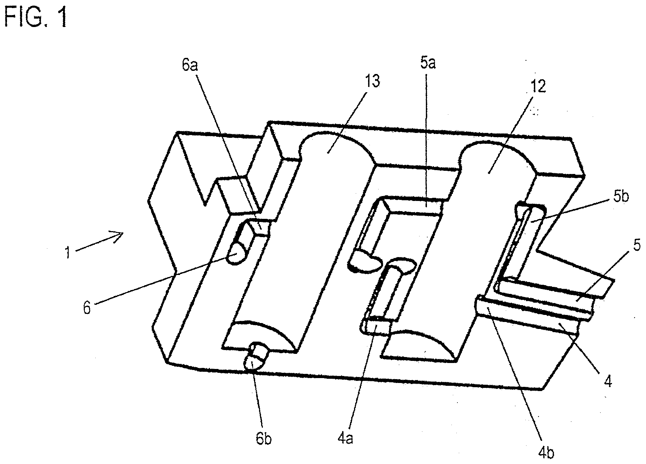

Zunächst ist unter Bezugnahme auf die Figuren 1 bis 3 ein erstes Ausführungsbeispiel erläutert.

Figur 1 zeigt ein Anwendungsbeispiel eines erfindungsgemäßen Fluidblocks.

Insbesondere ist in Figur 1 eine Schnittansicht eines erfindungsgemäßen Fluidblocks 1 gezeigt. Der Fluidblock 1 ist aus einem vorzugsweise transparenten Kunsttoffmaterial hergestellt. Der Fluidblock 1 kann durch Gussformen hergestellt werden. Der Fluidblock 1 kann aber auch durch ein mechanisches Bearbeiten wie z.B. Fräsen und Bohren hergestellt werden. Die Erfindung ist nicht auf ein spezifisches Herstellverfahren beschränkt.

Der Fluidblock 1 ist im vorliegenden Beispiel annähernd in Quaderform aufgebaut und besitzt eine proximale Seite an der linken Seite von Fig. 1 und eine distale Seite an der rechten Seite von Fig. 1. Der Fluidblock 1 hat eine Außenform, die an einen Innenraum eines nachstehend beschriebenen Endoskopbedienteils angepasst ist. Um einen sicheren Sitz im Endoskopbedienteil sicherzustellen, kann der Fluidblock 1 eine oder mehrere Erhebungen wie jene in Figur 1 an der zum Betrachter abgewandten Seite aufweisen. Die dem Fluidblock 1 abgewandte Stirnfläche der Erhebung(en) liegt dann an einer Innenwand des Endoskopbedienteils an.

Durch den Fluidblock 1 erstreckt sich zumindest ein Gasführungskanal 4 und zumindest ein Flüssigkeitsführungskanal 5. Darüber hinaus ist im vorliegenden Beispiel ein Arbeitskanal 6 im Fluidblock 1 vorgesehen.

Im Fluidblock 1 ist ein zylindrisches Sackloch 12 für ein nicht gezeigtes und nachstehend genauer beschriebenes Steuerventil so vorgesehen, dass das Sackloch 12 sowohl den Gasführungskanal 4 als auch den Flüssigkeitsführungskanal 5 schneidet. Das Steuerventil wird vom Anwender betätigt und blockiert und öffnet wahlweise sowohl den Gasführungskanal 4 als auch den Flüssigkeitsführungskanal 5.

Das Sackloch 12 unterteilt den Gasführungskanal 4 in einen proximalen Abschnitt 4a und einen distalen Abschnitt 4b, und unterteilt den Flüssigkeitsführungskanal 5 in einen proximaler Abschnitt 5a und einen distalen Abschnitt 5b. Die proximalen Abschnitte 4a und 5a führen zum Endoskopstecker hin und die distalen Abschnitte 4b und 5b führen zum Kathetheranschluss hin.

Der Fluidblock 1 besitzt somit eine Mündung des Gasführungskanals 4 und eine Mündung des Flüssigkeitsführungskanals 5 an einer Kathetheranschlussseite des Fluidblocks 1 und eine Mündung des Gasführungskanals 4 und eine Mündung des Flüssigkeitsführungskanals 5 an einer Endoskopsteckerseite des Fluidblocks 1. In Fig. 1 befinden sich die Mündung des distalen Abschnittes 4b des Gasführungskanals 4 und die Mündung des distalen Abschnittes 5b des Flüssigkeitsführungskanals 5 an der rechten Seite der Zeichnung. Anders ausgedrückt befinden sich die Mündung des distalen Abschnittes 4b des Gasführungskanals 4 und die Mündung des distalen Abschnittes 5b des Flüssigkeitsführungskanals 5 an der distalen Seite des Fluidblocks 1. Die Mündung des proximalen Abschnittes 4a des Gasführungskanals 4 und die Mündung des proximalen Abschnittes 5a des Flüssigkeitsführungskanals 5 an einer Endoskopsteckerseite sind in Fig. 1 nicht gezeigt. Sie können sich an der proximalen Seite des Fluidblocks 1 befinden. Die Erfindung ist nicht darauf beschränkt. Die Mündungen der Kanäle können auch an einer beliebigen anderen Seite des Fluidblocks 1 angeordnet werden.

Durch den Fluidblock 1 erstreckt sich darüber hinaus ein Arbeitskanal 6.

Im Fluidblock 1 ist ein zylindrisches Sackloch 13 für ein nicht gezeigtes und nachstehend genauer beschriebenes Arbeitskanalventil so vorgesehen, dass das Sackloch 13 den Arbeitskanal 6 schneidet. Vorzugsweise ist das Sackloch 12 zum Sackloch 13 parallel und benachbart angeordnet. Das Arbeitskanalventil ist somit vorzugsweise benachbart zum Steuerventil angeordnet. Das Arbeitskanalventil wird vom Anwender betätigt und blockiert und öffnet wahlweise den Arbeitskanal 6.

Das Sackloch 13 unterteilt den Arbeitskanal 6 in einen proximalen Abschnitt 6a und einen distalen Abschnitt 6b. Der proximale Abschnitt 6a führt zum Endoskopstecker hin und der distale Abschnitt 6b führt zum Kathetheranschluss hin.

Der Fluidblock 1 besitzt somit eine Mündung des Arbeitskanals 6 an einer Kathetheranschlussseite des Fluidblocks 1 und eine Mündung des Arbeitskanals 6 an einer Endoskopsteckerseite des Fluidblocks 1. Beide Mündungen sind in Fig. 1 nicht gezeigt. Die Mündungen des Arbeitskanals 6 können an einer beliebigen anderen Seite des Fluidblocks 1 angeordnet werden. Vorzugsweise befindet sich die Mündung des distalen Abschnittes 6b an der distalen Seite des Fluidblocks 1.

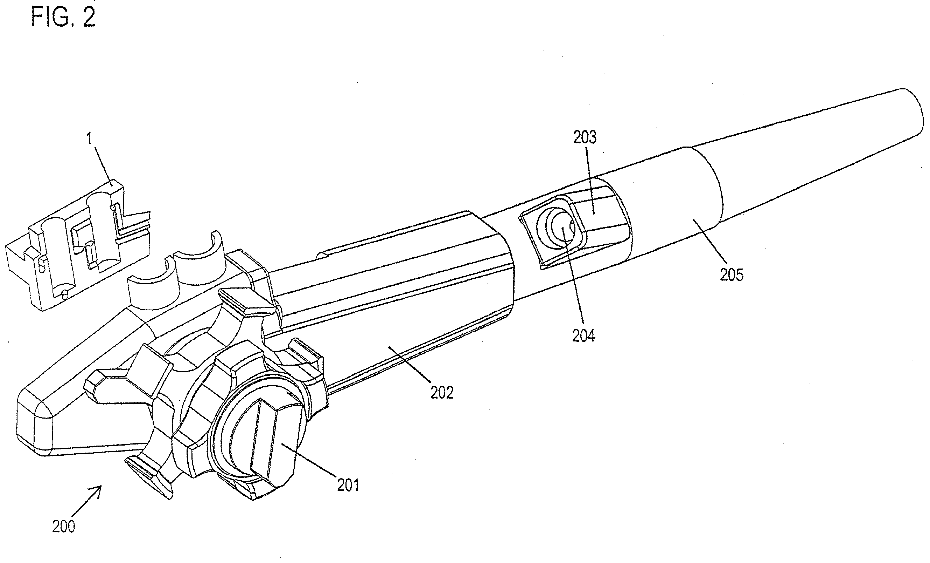

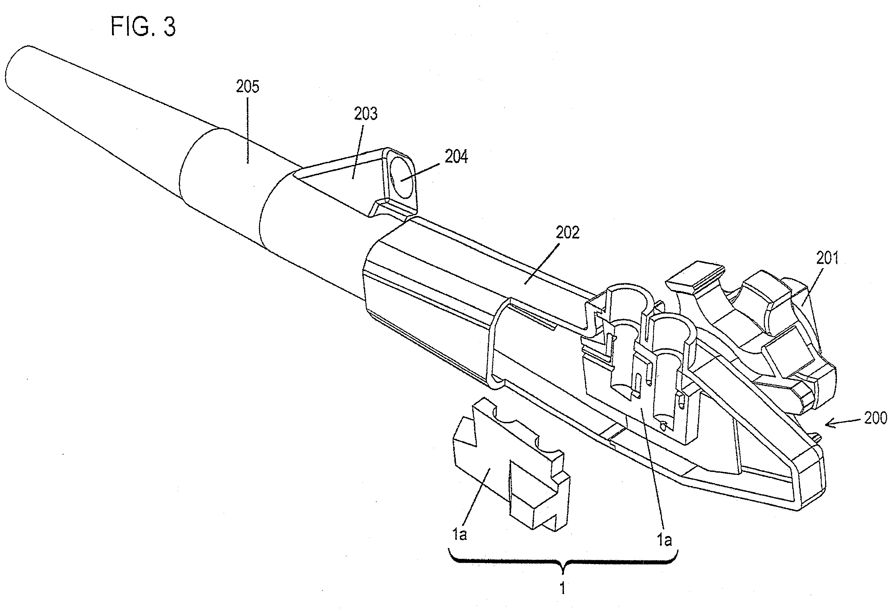

Figur 2 zeigt eine perspektivische Explosionsansicht eines Endoskopbedienteils mit dem Fluidblock von Fig. 1. Figur 3 zeigt eine perspektivische Explosionsansicht des Endoskopbedienteils mit dem Fluidblock von Fig. 2 unter Betrachtung von der Rückseite.

Ein Endoskop 200 hat ein Steuerelement 201 an einem Endoskopbedienteil 202. An der distalen Seite des Endoskopgriffes 202 erstreckt sich unter einem vorbestimmten Winkel, der 45° betragen kann, ein Zugangsanschluss 203 für ein Sekundärendoskop. Der vorbestimmte Winkel ist nicht auf 45° beschränkt.

Das Endoskop 200 besitzt distal vom Zugangsanschluss 203 für ein Sekundärendoskop einen Anschluss 205 für ein nicht gezeigtes Schlauchelement, d.h. einen Katheter. Im Endoskopbedienteil 202 verläuft ein in den Figuren 2 und 3 nicht gezeigter zentrisch vorgesehener Arbeitskanal, der in einem nicht gezeigten Schlauchelement des Endoskops weiterläuft. Von diesem Arbeitskanal zweigt eine Arbeitskanalabzweigung 204 in den Zugangsanschluss 203 ab. Im Zugangsanschluss 203 verläuft die Arbeitskanalabzweigung 204 ebenfalls zentrisch. Der innere Ausgang der Arbeitskanalabzweigung 204 mündet somit in den Arbeitskanal des Endoskops 200. Der Eingang der Arbeitskanalabzweigung 204 besitzt eine Öffnung an der proximalen Seite des Zugangsanschlusses 203. An dieser Öffnung ist ein Montagekörper für die Verbindung mit dem Sekundärendoskop drehsicher anbringbar.

Der Fluidblock 1 kann als ein einziger Fluidblock 1 aus einem Stück hergestellt sein. Wie dies in Fig. 3 gezeigt ist, kann der Fluidblock 1 auch aus zwei Fluidblockgegenstücken 1a bestehen, die miteinander z.B. durch Kleben oder Schweißen befestigt werden und somit den Fluidblock 1 ausbilden. Das linke Fluidblockgegenstück 1a in der Darstellung von Fig. 3 entspricht dem in Fig. 1 gezeigten aufgeschnittenen Fluidblock.

### Zweites Ausführungsbeispiel

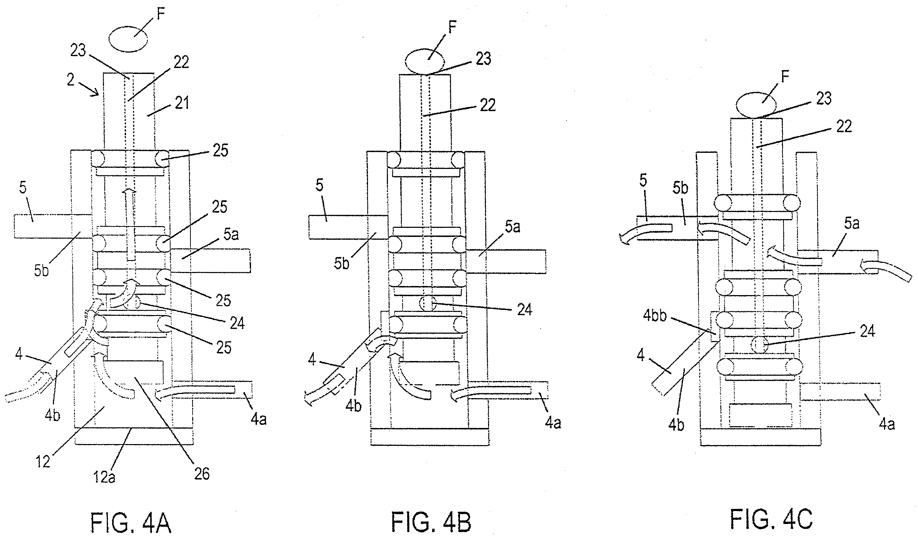

Zunächst ist unter Bezugnahme auf die Figuren 4 bis 6 ein zweites Ausführungsbeispiel erläutert.

Figur 4 zeigt eine schematische Schnittansicht eines Steuerventils zum Öffnen/Blockieren eines Gasführungskanals und eines Flüssigkeitsführungskanals, wobei Fig. 4A eine Luftöffnungsstellung zeigt, Fig. 4B eine Luftzuführstellung zeigt und Fig. 4C eine Spülstellung zeigt.

Im zweiten Ausführungsbeispiel ist ein Fluidblock vorgesehen, der ähnlich wie der Fluidblock 1 des ersten Ausführungsbeispiels aufgebaut ist.

Im Sackloch 12 ist ein in Figur 4 gezeigtes Steuerventil 2 so vorgesehen, dass es sowohl den Gasführungskanal 4 als auch den Flüssigkeitsführungskanal 5 schneidet. Das Steuerventil 4 wird vom Anwender betätigt und blockiert und öffnet wahlweise sowohl den Gasführungskanal 4 als auch den Flüssigkeitsführungskanal 5. Das Sackloch 12 hat einen Sacklochboden 12A.

Im Sackloch 12 münden der proximale Abschnitt 5a des Flüssigkeitsführungskanals 5, distale Abschnitt 5b des Flüssigkeitsführungskanals 5, der proximale Abschnitt 4a des Gasführungskanals 4 und der distale Abschnitt 4b des Gasführungskanals 4. Genauer gesagt stehen die Mündung des proximalen Abschnittes 5a des Flüssigkeitsführungskanals 5 und die Mündung des distalen Abschnittes 5b des Flüssigkeitsführungskanals 5 sich diametral gegenüber, sind aber in Längsrichtung des Sacklochs 12 zueinander versetzt, wie dies in Fig. 4A gezeigt ist. Außerdem stehen die Mündung des proximalen Abschnittes 4a des Gasführungskanals 4 und die Mündung des distalen Abschnittes 4b des Gasführungskanals 4 sich diametral gegenüber und sind in Längsrichtung des Sacklochs 12 zueinander versetzt. Unter Betrachtung vom Sacklochboden 12A aus münden die Kanalabschnitte in das Sackloch 12 in der Reihenfolge: proximaler Abschnitt 4a des Gasführungskanals 4, distaler Abschnitt 4b des Gasführungskanals 4, proximaler Abschnitt 5a des Flüssigkeitsführungskanals 5 und distaler Abschnitt 5b des Flüssigkeitsführungskanals 5.

Der Aufbau des Steuerventils 2 ist nachstehend beschrieben. Das Steuerventil 4 ist als ein Schieberventil aufgebaut.

Das Steuerventil 2 besitzt einen sich im zylinderartigen Schieberventilkörper des Steuerventils 2 erstreckenden Luftabgabekanal 22, dessen offenes Außenende 23 außerhalb des Fluidblocks 1 nach außen weist, und dessen Innenende 24 in der Gaszuführstellung des Steuerventils 2 mit dem Gasführungskanal 4 in Kommunikation steht. Vorzugsweise verläuft der Luftabgabekanal 22 zentrisch im Inneren des Schieberventilkörpers des Steuerventils 2. Der Luftabgabekanal 22 besitzt einen Außenabschnitt und einen Innenabschnitt. Der Außenabschnitt des Luftabgabekanals 22 mündet am Außenende 23 zur Außenseite und ist als Sackloch im Schieberventilkörper des Steuerventils 2 ausgebildet. Am inneren Ende des Sacklochs ist der Innenabschnitt des Luftabgabekanals 22 zur Außenumfangsfläche des Schieberventilkörpers vorzugsweise unter rechtem Winkel abgewinkelt. Das Ende des Innenabschnitts des Luftabgabekanals 22 an der Außenumfangsfläche des Schieberventilkörpers bildet das Innenende 24 des Luftabgabekanals 22. Das Innenende 24 des Luftabgabekanals 22 ist zur Innenumfangsfläche des Sacklochs 12 gerichtet und von diesem beabstandet.

Der Schieberventilkörper des Steuerventils 2 ist an seinem Außenumfang an geeigneten Positionen mit Dichtungen 25 versehen, deren Innenfläche am Außenumfang des Schieberventilkörpers dicht anliegt und deren Außenfläche am Innenumfang des Sacklochs 12 dicht anliegt. Die Dichtungen 25 sind relativ zum Steuerventil 2 fixiert und unbeweglich.

Eine erste Dichtung 25 sitzt in herausbewegter Stellung des Steuerventils 2 (siehe Fig. 4A) am Außenumfang des Schieberventilkörpers derart, dass die erste Dichtung noch im Sackloch 12 sitzt. Im Betrieb wird das Steuerventil 2 nicht so weit aus dem Sackloch 12 herausbewegt, dass die erste Dichtung 25 das Sackloch 12 verlässt. Zwischen der ersten Dichtung 25 und dem in Fig. 4 nach oben weisenden Ende des Schieberventilkörpers, das das Außenende 23 des Luftabgabekanals 22 aufweist, erstreckt sich ein vorragender Abschnitt 21 des Schieberventilkörpers.

Eine zweite Dichtung 25 sitzt in herausbewegter Stellung des Steuerventils 2 beabstandet zur ersten Dichtung 25 an einer Position am Außenumfang des Schieberventilkörpers so, dass am Sacklochinnenumfang der distale Abschnitt 5b des Flüssigkeitsführungskanals 5 zwischen der ersten Dichtung 25 und der zweiten Dichtung 25 mündet.

Eine dritte Dichtung 25 sitzt in herausbewegter Stellung des Steuerventils 2 beabstandet zur zweiten Dichtung 25 an einer Position am Außenumfang des Schieberventilkörpers so, dass am Sacklochinnenumfang der proximale Abschnitt 5a des Flüssigkeitsführungskanals 5 zwischen der zweiten Dichtung 25 und der dritten Dichtung 25 mündet.

Eine vierte Dichtung 25 sitzt in herausbewegter Stellung des Steuerventils 2 beabstandet zur ersten Dichtung 25 an einer Position am Außenumfang des Schieberventilkörpers so, dass am Außenumfang des Schieberventilkörpers das Innenende 24 des Luftabgabekanals 22 zwischen der dritten Dichtung 25 und der vierten Dichtung 25 mündet.

Die herausbewegte Stellung des Steuerventils 2 ist in den Figuren 4A und 4B gezeigt.

Wie dies in Fig. 4A gezeigt ist, ist in herausbewegter Stellung des Steuerventils 2 der Innenendabschnitt des Schieberventilkörpers des Steuerventils 2 vom Sacklochboden 12A des Sacklochs 12 beabstandet.

Vorzugsweise besitzt das Sackloch 12 eine Vertiefung 4bb an seiner Umfangswand an der Stelle, an der der distale Abschnitt 4b des Gasführungskanals 4 in das Sackloch 12 mündet. Die Länge der Vertiefung 4bb in der Längsrichtung des Sacklochs 12 ist länger als der Durchmesser der vierten Dichtung 25. In herausbewegter Stellung des Steuerventils 2 sitzt die vierte Dichtung 25 an der Vertiefung 4bb so, dass die vierte Dichtung 25 zum - in der Längsrichtung des Sacklochs 12 gesehen - nach außen gerichteten Ende und zum nach innen gerichteten Ende der Vertiefung 4bb beabstandet ist. Somit ist über die Vertiefung 4bb eine Fluidbewegung zwischen dem Bereich oberhalb der vierten Dichtung 25 (der Bereich zwischen der dritten Dichtung 25 und der vierten Dichtung 25) und dem Bereich unterhalb der vierten Dichtung 25 (der Bereich zwischen dem Sacklochboden 12A und der vierten Dichtung 25) möglich.

In der hineinbewegten Stellung des Steuerventils 2 sitzt die erste Dichtung 25 sitzt zwischen dem nach außen gerichteten offenen Ende des Sacklochs 12 und dem distalen Abschnitt 5b des Flüssigkeitsführungskanals 5.

In der hineinbewegten Stellung des Steuerventils 2 sitzt die zweite Dichtung 25 unterhalb des proximalen Abschnitts 5a des Flüssigkeitsführungskanals 5, sitzt die dritte Dichtung 25 am nach außen gerichteten Ende der Vertiefung 4bb am distalen Abschnitt 4b des Gasführungskanals 4, und sitzt die vierte Dichtung 25 zwischen dem proximalen Abschnitt 4a des Gasführungskanals 4 und dem distalen Abschnitt 4b des Gasführungskanals 4.

Die hineinbewegte Stellung des Steuerventils 2 ist in Figur 4C gezeigt.

Das Steuerventil 2 ist somit ein in zwei Schaltstellungen verschiebbares Schieberventil. Die herausbewegte Stellung des Steuerventils 2 ist eine Gaszuführstellung. Die hineinbewegte Stellung des Steuerventils 2 ist eine Flüssigkeitszuführstellung. Das Steuerventil 2 kann zwischen der Gaszuführstellung und der Flüssigkeitszuführstellung durch Verschieben geschaltet werden.

In der Gaszuführstellung befindet sich die vierte Dichtung 25 an einer Position, unterhalb der eine Kommunikation zwischen dem proximalen Abschnitt 4a des Gasführungskanals 4 und dem distalen Abschnitt 4b des Gasführungskanals 4 ermöglicht ist. Der Gasführungskanal 4 ist somit offen. In der Gaszuführstellung befindet sich die zweite Dichtung 25 unterhalb des distalen Abschnitt 5b des Flüssigkeitsführungskanals 5 und oberhalb des proximalen Abschnitts 5a des Flüssigkeitsführungskanals 5 und unterbindet die Kommunikation zwischen ihnen. Der Flüssigkeitsführungskanal 5 ist somit geschlossen.

In der Gaszuführstellung sind zwei Betriebsmodi möglich.

Fig. 4A zeigt den Luftabgabemodus. Da im Luftabgabemodus die vierte Dichtung an der Vertiefung 4bb so sitzt, dass eine Fluidbewegung zwischen dem Bereich unterhalb der vierten Dichtung 25 und dem Bereich oberhalb der vierten Dichtung 25 möglich ist und zwischen der dritten Dichtung 25 und der vierten Dichtung 25 sich das Innenende 24 des Luftabgabekanals 22 befindet, kann Gas, wie z.B. Luft, von dem distalen Abschnitt 4b des Gasführungskanals 4 über den Luftabgabekanal 22 nach außen abgegeben werden. Selbst eine Zufuhr von Gas aus dem proximalen Abschnitt 4a des Gasführungskanals 4 behindert nicht die Gasabgabe von dem distalen Abschnitt 4b des Gasführungskanals 4 über den Luftabgabekanal 22 nach außen.

Fig. 4B zeigt den Aufblasmodus. Der vorragende Abschnitt 21 des Schieberventilkörpers des Steuerventils 2 ragt zur Außenseite vor und besitzt die äußere Öffnung 23 des Luftabgabekanals 22. Der Anwender kann die äußere Öffnung 23 des Luftabgabekanals 22 durch einen Finger F, wie z.B. den Daumen, verschließen. Der Schieberventilkörper des Steuerventils 2 sitzt über die Dichtungen 25 und deren Reibschluss derart verschiebesicher im Sackloch 12, dass, wenn der Finger F des Anwenders gegen die äußere Öffnung 23 des Luftabgabekanals 22 gedrückt wird, der Schieberventilkörper des Steuerventils 2 nicht in das Sackloch 12 hinein geschoben wird.

Erst durch ein gezieltes Überwinden des Reibschlusses der Dichtungen 25 am Innenumfang des Sackloches 12 kann der Schieberventilkörper des Steuerventils 2 von der Gaszuführstellung in die Flüssigkeitszuführstellung geschoben werden.

Fig. 4C zeigt die Flüssigkeitszuführstellung. Die vierte Dichtung 25 befindet sich zwischen dem proximalen Abschnitt 4a des Gasführungskanals 4 und dem distalen Abschnitt 4b des Gasführungskanals 4. Der Gasführungskanal 4 ist somit unterbrochen. Da sich die Mündung des distalen Abschnittes 4b des Gasführungskanals 4 unterhalb der dritte Dichtung 25 befindet und eine Fluidkommunikation somit zwischen dem distalen Abschnitt 4b des Gasführungskanals 4 und dem Innenende 24 des Luftabgabekanals 22 möglich ist, kann Gas immer noch über den Luftabgabekanal 22 nach außen abgegeben werden. Zwischen der ersten Dichtung 25 und der zweiten Dichtung 25 stehen der proximale Abschnitt 5a des Flüssigkeitsführungskanals 5 und der distale Abschnitt 5b des Flüssigkeitsführungskanals 5 in Fluidkommunikation. Der Flüssigkeitsführungskanal 5 ist somit geöffnet.

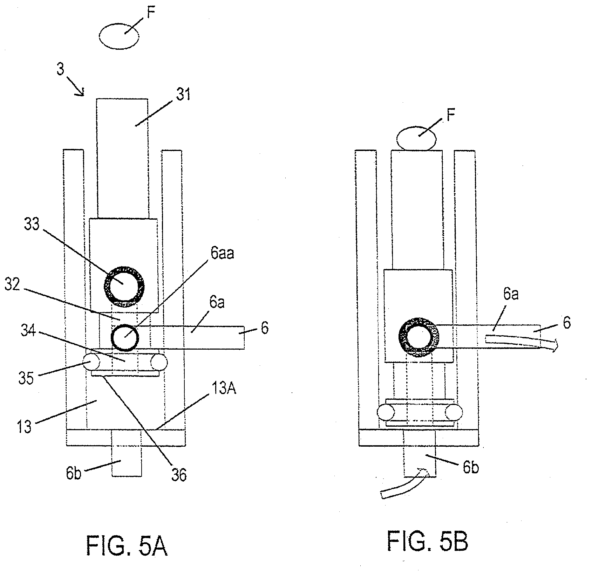

Figur 5 zeigt eine schematische Schnittansicht eines Arbeitskanalventils, wobei Fig. 5A eine Stellung zeigt, in der der Arbeitskanal geschlossen ist, und Fig. 5B eine Stellung zeigt, in der der Arbeitskanal geöffnet ist.

Das Sackloch 13 von Figur 5 hat einen Sacklochboden 13A und weist zentrisch am Sacklochboden 13A eine Mündung des sich im vorliegenden Beispiel nach unten erstreckenden distalen Abschnittes 6b des Arbeitskanals 6 auf. Der proximale Abschnitt 6a des Arbeitskanals 6 mündet an der Innenumfangsfläche des Sacklochs 13. Der proximale Abschnitt 6a besitzt eine Mündung 6aa, wie die in Fig. 5 angedeutet ist.

Im Sackloch 13 ist ein in Figur 5 gezeigtes Arbeitskanalventil 3 so vorgesehen, dass es den Arbeitskanal 6 schneidet. Das Arbeitskanalventil 3 ist benachbart zum Steuerventil 2 so im Fluidblock 1 angeordnet, dass es von der Außenseite des Fluidblocks 1 betätigbar ist, wobei das Arbeitskanalventil 3 zwischen einer Stellung, in der der Arbeitskanal 6 geöffnet ist, und einer Stellung, in der der Arbeitskanal 6 geschlossen ist, schaltbar ist. Das Arbeitskanalventil 3 wird vom Anwender betätigt und blockiert und öffnet wahlweise den Arbeitskanal 6.

Der genaue Aufbau des Arbeitskanalventils 3 ist nachstehend beschrieben.

Das Arbeitskanalventil 3 ist ebenfalls als ein Schieberventil aufgebaut. Das Arbeitskanalventil 3 hat einen zylinderartigen Schieberventilkörper.

Das Arbeitskanalventil 3 besitzt einen im zylinderartigen Schieberventilkörper des Arbeitskanalventils 3 angeordneten Innenkanal 32. Der Innenkanal 32 besitzt einen Eingangsabschnitt und einen Ausgangsabschnitt. Der Eingangsabschnitt des Innenkanals 32 ist als Sackloch ausgebildet und verläuft vorzugsweise zentrisch im Inneren des Schieberventilkörpers des Arbeitskanalventils 3 und hat eine Eingangsöffnung 34 an der Sacklochbodenseite des Schieberventilkörpers. Am inneren Ende des Eingangsabschnittes des Innenkanals 32 geht der Innenkanals 32 in einen Ausgangsabschnitt über, der zur Außenumfangsfläche des Schieberventilkörpers vorzugsweise unter rechtem Winkel abgewinkelt ist. Das Ende des Ausgangsabschnittes des Innenkanals 32 an der Außenumfangsfläche des Schieberventilkörpers bildet die Abgabeöffnung 33 des Innenkanals 32. Die Abgabeöffnung 33 des Innenkanals 32 ist zur Innenumfangsfläche des Sacklochs 13 gerichtet und von diesem beabstandet.

Der Schieberventilkörper des Arbeitskanalventils 3 ist an seinem Außenumfang an einer geeigneten Position am unteren Ende (Sacklochbodenende) des Schieberventilkörpers mit einer Dichtung 35 versehen, deren Innenfläche am Außenumfang des Schieberventilkörpers dicht anliegt und deren Außenfläche am Innenumfang des Sacklochs 13 dicht anliegt. Die Dichtung 35 ist relativ zum Arbeitskanalventil 3 fixiert und unbeweglich. Die Dichtung 35 befindet sich bei im Sackloch 13 eingebautem Arbeitskanalventil 3 zwischen dem distalen Abschnitt 6b des Arbeitskanals 6 und dem proximalen Abschnitt 6a des Arbeitskanals 6.

Das Arbeitskanalventil 3 ist somit ein in zwei Schaltstellungen verschiebbares Schieberventil. Die herausbewegte Stellung des Arbeitskanalventils 3 ist eine geschlossene Stellung. Die hineinbewegte Stellung des Arbeitskanalventils 3 ist eine geöffnete Stellung. Das Arbeitskanalventil 3 kann zwischen der geschlossenen Stellung und der geöffneten Stellung durch Verschieben geschaltet werden. In der geschlossenen Stellung ist der Arbeitskanal 6 unterbrochen. In der geöffneten Stellung ist der Arbeitskanal 6 offen, und es kann durch den Arbeitskanal 6 abgesaugt werden.

Fig. 5A zeigt die geschlossene Stellung. In der geschlossenen Stellung befindet sich die zur Innenumfangsfläche des Sacklochs 13 weisende Abgabeöffnung 33 des Innenkanals 32 nicht in Gegenüberlage zu der Mündung 6aa des proximalen Abschnittes 6a des Arbeitskanals 6. Die Abgabeöffnung 33 des Innenkanals 32 besitzt beispielsweise eine Mündungsdichtung, die den Rand der Abgabeöffnung 33 des Innenkanals 32 an der Innenumfangsfläche des Sacklochs 13 abdichtet.

Durch ein Hineinschieben des Arbeitskanalventils 3 zum Sacklochboden 13A hin und ein damit verbundenes Überwinden des Reibschlusses der Dichtung 35 am Innenumfang des Sackloches 13 kann der Schieberventilkörper des Arbeitskanalventils 3 von der geschlossenen Stellung in die geöffnete Stellung geschoben werden.

Fig. 5B zeigt die geöffnete Stellung. In der geöffneten Stellung liegt die untere Stirnfläche des Schieberventilkörpers des Arbeitskanalventils 3 am Sacklochboden 13A an, wobei in dieser Stellung die zur Innenumfangsfläche des Sacklochs 13 weisende Abgabeöffnung 33 des Innenkanals 32 der Mündung 6aa des proximalen Abschnittes 6a des Arbeitskanals 6 gegenüberliegt. Zwischen dem proximalen Abschnitt 6a des Arbeitskanals 6 und dem distalen Abschnitt 6b des Arbeitskanals 6 ist somit über den Innenkanal 32 eine Fluidkommunikation möglich. Der Arbeitskanal 6 ist somit geöffnet.

Vorzugsweise ist das Steuerventil 2 und/oder das Arbeitskanalventil 3 ein Wegwerfventil zur Einmalverwendung. Vorzugsweise ist das Steuerventil 2 und/oder das Arbeitskanalventil 3 aus geformten Zweikomponenten-Kunststoffen hergestellt. Die Ventile bilden somit kein Risiko einer Kontamination und ein kostenspieliger Reinigungsvorgang entfällt.

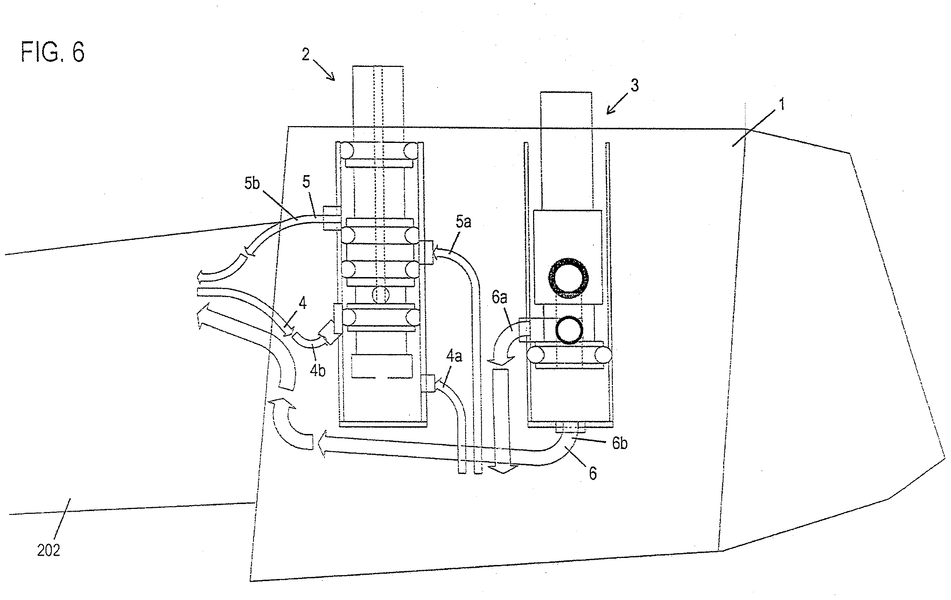

Figur 6 zeigt ein Anwendungsbeispiel eines erfindungsgemäßen Fluidblocks mit dem Steuerventil und dem Arbeitskanalventil der Figuren 4 und 5.

Das Steuerventil 2 und das Arbeitskanalventil 3 sind parallel und benachbart zueinander im Fluidblock 1 in ihren Betriebspositionen vorgesehen. In Figur 6 ist darüber hinaus der Verlauf der Kanäle im Fluidblock 1 angedeutet. Vom Steuerventil 2 führen der distale Abschnitt 4b des Gasführungskanals 4 und der distale Abschnitt 5b des Flüssigkeitsführungskanals 5 und vom Arbeitskanalventil 3 führt der distale Abschnitt 6b des Arbeitskanals 6 im Fluidblock 1 zur distalen Seite. An der distalen Seite besitzt der Fluidblock 1 die jeweiligen Anschlüsse der Kanäle, die eine jeweilige Kanalweiterführung durch das Endoskopbedienteil 202 zum Katheter und zum distalen Endoskopende ermöglichen. Vom Steuerventil 2 führen der proximale Abschnitt 4a des Gasführungskanals 4 und der proximale Abschnitt 5a des Flüssigkeitsführungskanals 5 und vom Arbeitskanalventil 3 führt der proximale Abschnitt 6a des Arbeitskanals 6 im Fluidblock 1 zur proximalen Seite. An der proximalen Seite besitzt der Fluidblock 1 die jeweiligen Anschlüsse der Kanäle, die eine jeweilige Kanalweiterführung zum proximalen Endoskopstecker ermöglichen.

Der Fluidblock 1 besitzt somit lediglich die proximalen und distalen Kanalanschlüsse. Daher ist der Fluidblock 1 kostengünstig z.B. durch ein Formverfahren herstellbar. Der Zusammenbau ist einfach. Die Wartungsfreundlichkeit des Endoskopbedienteils 202 ist verbessert.

Der Fluidblock 1 kann im als Griffstück ausgebildeten Endoskopbedienteil 202 so angeordnet werden, dass der Fluidblock 1 von einem Gehäuseabschnitt des Endoskopbedienteil 202 umgeben ist. Dieser Gehäuseabschnitt des Endoskopbedienteil 202 hat eine Innenform, an die der Fluidblock 1 angepasst ist, und besitzt Öffnungen, durch die die vorragenden Abschnitte 21, 31 der Ventile 2 und 3 passieren.

Der Fluidblock 1 kann auch selbst das Griffstück des Endoskopbedienteil 202 ausbilden und entsprechend geformt sein. Dann kann auf ein zusätzliches Gehäuse verzichtet werden.

### Alternativen

Im ersten und zweiten Beispiel sind ein Gasführungskanal 4, ein Flüssigkeitsführungskanal 5 und ein Arbeitskanal 6 im Fluidblock 1 vorgesehen. Die Anzahl dieser Kanäle ist nicht beschränkt. Es können in einem Fluidblock mehrere Gasführungskanäle und/oder mehrere Flüssigkeitsführungskanäle und/oder mehrere Arbeitskanäle vorgesehen sein.

Im Fluidblock können außer dem Gasführungskanal 4, dem Flüssigkeitsführungskanal 5 und dem Arbeitskanal 6 ein oder mehrere Kanäle für eine oder mehrere elektrische Leitung(en) für ein oder mehrere elektronische Instrumente am distalen Endoskopschlauchende, und Kanäle für Zugseile zur Deflectingsteuerung vorgesehen sein.

Im ersten und zweiten Beispiel sind im Fluidblock 1 ein Sackloch 12 für das Steuerventil 2 und ein Sackloch 13 für ein Arbeitskanalventil 3 vorgesehen. In einer Alternative können die Löcher im Fluidblock, die für das Steuerventil 2 und das Arbeitskanalventil 3 vorgesehen sind, auch durchgehende Löcher sein. Eine Ventilanschlagfläche, wie sie durch den Sacklochboden in den Beispielen vorgesehen wird, kann durch eine Querschnittsverengung im jeweiligen Loch oder durch einen eingesetzten Stift oder eine eingesetzte Scheibe etc. vorgesehen werden. In einer weiteren Alternative können durchgehende Löcher ohne Ventilanschlagfläche vorgesehen sein. In diesem Fall können die in den Löchern sitzenden Ventile ihre Ventilstellungen ausschließlich von der Bedienerseite erlangen.

Die Positionen der Kanalabschnitte 4a, 4b, 5a, 5b, 6a und 6b in den Sacklöchern können nach Bedarf und unter entsprechender Anpassung der jeweiligen Ventile abgewandelt werden.

Die Löcher 12 und 13 für die Ventile sind in den Ausführungsbeispielen zylindrisch. Die Löcher für die Ventile können eine beliebige Form haben. Ventile mit eine beliebigen Außenform können an diese Ventilaußenform angepasste Querschnitte und Formen im Fluidblock bedingen.

### Bezugszeichenliste

1 Fluidblock
1a Fluidblockgegenstücke
2 Steuerventil
3 Arbeitskanalventil
4 Gasführungskanal
4a proximaler Abschnitt
4b distaler Abschnitt
4bb Vertiefung
5 Flüssigkeitsführungskanal
5a proximaler Abschnitt
5b distaler Abschnitt
6 Arbeitskanal
6a proximaler Abschnitt
6aa Mündung
6d distaler Abschnitt
12 Sackloch für Steuerventil
12A Sacklochboden
13 Sackloch für Arbeitskanalventil
13A Sacklochboden
21 vorragender Abschnitt
22 Luftabgabekanal
23 äußere Öffnung des Luftabgabekanals
24 innere Öffnung des Luftabgabekanals
25 Dichtung
26 Ventilendabschnitt
31 vorragender Abschnitt
32 Innenkanal
33 Abgabeöffnung des Innenkanals
34 Eingangsöffnung des Innenkanals
35 Dichtung
36 Ventilendabschnitt
200 Endoskop
201 Steuerelement des Endoskops
202 Endoskopbedienteil
203 Zugangsanschluss für ein Sekundärendoskop
204 Arbeitskanalabzweigung im Zugangsanschluss
205 Anschluss für Schlauchelement
F Finger

## Patentansprüche

1. Fluidblock (1) für ein Endoskopbedienteil (202), mit
zumindest einem im Inneren des Fluidblocks (1) vorgesehenen Gasführungskanal (4) mit einer Eingangsöffnung und einer Ausgangsöffnung,
zumindest einem im Inneren des Fluidblocks (1) vorgesehenen Flüssigkeitsführungskanal (5) mit einer Eingangsöffnung und einer Ausgangsöffnung,
und zumindest einem Steuerventil (2) zum Öffnen/Blockieren des zumindest einen Gasführungskanals (4) und des zumindest einen Flüssigkeitsführungskanals (5),
**dadurch gekennzeichnet, dass**
der Fluidblock (1) aus zwei Fluidblockgegenstücken (1a) besteht, die zum Ausbilden des Fluidblocks (1) aneinander befestigt sind.

2. Fluidblock (1) gemäß Anspruch 1, wobei
die beiden Fluidblockgegenstücke (1a) jeweils den Gasführungskanal (4), den Flüssigkeitsführungskanal (5) und das Steuerventil (2) schneiden, und zum Ausbilden des Fluidblocks (1) aneinander befestigt sind.

3. Fluidblock (1) gemäß Anspruch 1 oder 2, wobei
die beiden Fluidblockgegenstücke (1a) durch Kleben oder Schweißen aneinander befestigt sind.

4. Fluidblock (1) gemäß einem der Ansprüche 1 bis 3, wobei
das Steuerventil (2) so im Fluidblock (1) angeordnet ist, dass es von der Außenseite des Fluidblocks (1) betätigbar ist.

5. Fluidblock (1) gemäß einem der Ansprüche 1 bis 4, wobei der Fluidblock (1) aus transparentem Kunststoff hergestellt ist.

6. Fluidblock (1) gemäß einem der Ansprüche 1 bis 5, wobei
das Steuerventil (2) in dem zumindest einen Gasführungskanal (4) und in dem zumindest einen Flüssigkeitsführungskanal (5) angeordnet ist,
wobei das Steuerventil (2) zwischen einer Gaszuführstellung, in der das Steuerventil (2) den Gasführungskanal (4) öffnet, und einer Flüssigkeitszuführstellung, in der das Steuerventil (2) den Flüssigkeitsführungskanal (5) öffnet, schaltbar ist.

7. Fluidblock (1) gemäß Anspruch 6, wobei
das Steuerventil (2) ein in zwei Schaltstellungen verschiebbares Schieberventil ist, wobei in der Gaszuführstellung das Steuerventil (2) den Gasführungskanal (4) öffnet und den Flüssigkeitsführungskanal (5) schließt, und in der Flüssigkeitszuführstellung das Steuerventil (2) den Flüssigkeitsführungskanal (5) öffnet und den Gasführungskanal (4) schließt;
wobei das Steuerventil (2) einen sich im Körper des Steuerventils (2) erstreckenden Luftabgabekanal (22) aufweist, dessen offenes Außenende (23) außerhalb des Fluidblocks (1) nach außen weist, und dessen Innenende (24) in der Gaszuführstellung des Steuerventils (2) mit dem Gasführungskanal (4) in Kommunikation steht.

8. Fluidblock (1) gemäß Anspruch 7, wobei
wobei in der Flüssigkeitszuführstellung des Steuerventils (2) eine Kommunikation zwischen einem distalen Abschnitt (4b) des Gasführungskanals (4) und einem Innenende (24) des Luftabgabekanals (22) möglich ist

9. Fluidblock (1) gemäß einem der Ansprüche 1 bis 8, mit
einem im Inneren des Fluidblocks (1) vorgesehenen Arbeitskanal (6) mit einer Eingangsöffnung und einer Ausgangsöffnung.

10. Fluidblock (1) gemäß Anspruch 9, wobei
benachbart zum Steuerventil (2) ein Arbeitskanalventil (3) so im Fluidblock (1) angeordnet ist, dass es von der Außenseite des Fluidblocks (1) betätigbar ist, wobei das Arbeitskanalventil (3) zwischen einer Stellung, in der der Arbeitskanal (6) geöffnet ist, und einer Stellung, in der der Arbeitskanal (6) geschlossen ist, schaltbar ist.

11. Fluidblock (1) gemäß einem der Ansprüche 1 bis 10, wobei
das Steuerventil (2) und/oder das Arbeitskanalventil (3) ein Wegwerfventil zur Einmalverwendung ist.

12. Fluidblock (1) gemäß einem der Ansprüche 1 bis 11, mit
einem Kanal für zumindest eine elektrische Leitung für ein elektronisches Instrument am distalen Endoskopschlauchende, und
Kanälen für Zugseile zur Endoskopbiegesteuerung.

13. Endoskop mit einem Endoskopbedienteil (202) als Griffstück, wobei das Griffstück durch den Fluidblock (1) gemäß einem der Ansprüche 1 bis 12 gebildet ist, wobei eines der zwei Fluidblockgegenstücke (1a) in einem Griffstückteil integriert ist.

14. Endoskop mit einem Endoskopbedienteil (202) als Griffstück, wobei das Griffstück ein Gehäuse aufweist, in dem der Fluidblock (1) gemäß einem der Ansprüche 1 bis 12 angeordnet ist.

## Claims

1. Fluid block (1) for an endoscope control part (202), having
at least one gas-guiding channel (4), which is provided in the interior of the fluid block (1) and has an inlet opening and an outlet opening,
at least one liquid-guiding channel (5), which is provided in the interior of the fluid block (1) and has an inlet opening and an outlet opening,
and at least one control valve (2) for opening/blocking the at least one gas-guiding channel (4) and the at least one liquid-guiding channel (5),
**characterized in that**
the fluid block (1) consists of two fluid-block mating pieces (1a) which are fastened to one another for the purpose of forming the fluid block (1).

2. Fluid block (1) according to Claim 1, wherein
the two fluid-block mating pieces (1a) each cut the gas-guiding channel (4), the liquid-guiding channel (5) and the control valve (2), and are fastened to one another for the purpose of forming the fluid block (1).

3. Fluid block (1) according to Claim 1 or 2, wherein the two fluid-block mating pieces (1a) are fastened to one another by adhesive bonding or welding.

4. Fluid block (1) according to one of Claims 1 to 3,
wherein
the control valve (2) is arranged in the fluid block (1) such that it is able to be actuated from outside the fluid block (1).

5. Fluid block (1) according to one of Claims 1 to 4,
wherein
the fluid block (1) is manufactured from transparent plastic.

6. Fluid block (1) according to one of Claims 1 to 5,
wherein
the control valve (2) is arranged in the at least one gas-guiding channel (4) and in the at least one liquid-guiding channel (5),
wherein the control valve (2) is able to be switched between a gas supply position, in which the control valve (2) opens the gas-guiding channel (4), and a liquid supply position, in which the control valve (2) opens the liquid-guiding channel (5).

7. Fluid block (1) according to Claim 6, wherein
the control valve (2) is a slide valve which is able to be displaced into two switching positions, wherein, in the gas supply position, the control valve (2) opens the gas-guiding channel (4) and closes the liquid-guiding channel (5), and in the liquid supply position, the control valve (2) opens the liquid-guiding channel (5) and closes the gas-guiding channel (4);
wherein the control valve (2) has an air discharge channel (22) extending in the body of the control valve (2), whose open outer end (23) outside the fluid block (1) points outwards and whose inner end (24) is in communication with the gas-guiding channel (4) in the gas supply position of the control valve (2).

8. Fluid block (1) according to Claim 7, wherein,
in the liquid supply position of the control valve (2), communication between a distal section (4b) of the gas-guiding channel (4) and an inner end (24) of the air discharge channel (22) is possible.

9. Fluid block (1) according to one of Claims 1 to 8, having
a working channel (6), which is provided in the interior of the fluid block (1) and has an inlet opening and an outlet opening.

10. Fluid block (1) according to Claim 9, wherein, adjacent to the control valve (2), a working-channel valve (3) is arranged in the fluid block (1) such that it is able to be actuated from outside the fluid block (1), wherein the working-channel valve (3) is able to be switched between a position in which the working channel (6) is open and a position in which the working channel (6) is closed.

11. Fluid block (1) according to one of Claims 1 to 10, wherein
the control valve (2) and/or the working-channel valve (3) are/is a disposable valve for single use.

12. Fluid block (1) according to one of Claims 1 to 11, having
a channel for at least one electrical line for an electronic instrument at the distal endoscope tube end, and
channels for tension cables for endoscope bending control.

13. Endoscope having an endoscope control part (202) as a gripping piece, wherein the gripping piece is formed by the fluid block (1) according to one of Claims 1 to 12, wherein one of the two fluid-block mating pieces (1a) is integrated in a gripping-piece part.

14. Endoscope having an endoscope control part (202) as a gripping piece, wherein the gripping piece has a housing in which the fluid block (1) according to one of Claims 1 to 12 is arranged.

## Revendications

1. Bloc fluidique (1) pour un organe de commande d'un endoscope (202), comprenant
au moins un conduit de passage de gaz (4) prévu à l'intérieur du bloc fluidique (1) avec une ouverture d'entrée et une ouverture de sortie,
au moins un conduit de passage de liquide (5) prévu à l'intérieur du bloc fluidique (1) avec une ouverture d'entrée et une ouverture de sortie,
et au moins une soupape de commande (2) pour ouvrir/fermer l'au moins un conduit de passage de gaz (4) et l'au moins un conduit de passage de liquide (5),
**caractérisé en ce que**
le bloc fluidique (1) se compose de deux organes conjugués de bloc fluidique (1a) qui sont fixés l'un à l'autre pour constituer le bloc fluidique (1).

2. Bloc fluidique (1) selon la revendication 1, dans lequel
les deux organes conjugués de bloc fluidique (1a) intersectent à chaque fois le conduit de passage de gaz (4), le conduit de passage de liquide (5) et la soupape de commande (2), et sont fixés l'un à l'autre pour constituer le bloc fluidique (1).

3. Bloc fluidique (1) selon la revendication 1 ou 2, dans lequel les deux organes conjugués de bloc fluidique (1a) sont fixés l'un à l'autre par collage et/ou soudage.

4. Bloc fluidique (1) selon l'une quelconque des revendications 1 à 3, dans lequel
la soupape de commande (2) est disposée dans le bloc fluidique (1) de manière à pouvoir être commandée depuis le côté extérieur du bloc fluidique (1).

5. Bloc fluidique (1) selon l'une quelconque des revendications 1 à 4, le bloc fluidique (1) étant fabriqué à partir de matière plastique transparente.

6. Bloc fluidique (1) selon l'une quelconque des revendications 1 à 5, dans lequel
la soupape de commande (2) est disposée dans l'au moins un conduit de passage de gaz (4) et dans l'au moins un conduit de passage de liquide (5),
la soupape de commande (2) pouvant être commutée entre une position d'alimentation en gaz dans laquelle la soupape de commande (2) ouvre le conduit de passage de gaz (4) et une position d'alimentation en liquide dans laquelle la soupape de commande (2) ouvre le conduit de passage de liquide (5).

7. Bloc fluidique (1) selon la revendication 6, dans lequel
la soupape de commande (2) est une soupape à tiroir pouvant être coulissée dans deux positions de commutation, dans la position d'alimentation en gaz, la soupape de commande (2) ouvrant le conduit de passage de gaz (4) et fermant le conduit de passage de liquide (5), et dans la position d'alimentation en liquide, la soupape de commande (2) ouvrant le conduit de passage de liquide (5) et fermant le conduit de passage de gaz (4) ;
la soupape de commande (2) présentant un conduit de distribution d'air (22) s'étendant dans le corps de la soupape de commande (2), dont l'extrémité extérieure ouverte (23) est orientée vers l'extérieur à l'extérieur du bloc fluidique (1), et dont l'extrémité intérieure (24), dans la position d'alimentation en gaz de la soupape de commande (2), est en communication avec le conduit de passage de gaz (4).

8. Bloc fluidique (1) selon la revendication 7, dans lequel
dans la position d'alimentation en liquide de la soupape de commande (2), une communication est possible entre une portion distale (4b) du conduit de passage de gaz (4) et une extrémité intérieure (24) du conduit de distribution d'air (22).

9. Bloc fluidique (1) selon l'une quelconque des revendications 1 à 8, comprenant
un conduit de travail (6) prévu à l'intérieur du bloc fluidique (1), avec une ouverture d'entrée et une ouverture de sortie.

10. Bloc fluidique (1) selon la revendication 9, dans lequel une soupape de conduit de travail (3) est disposée dans le bloc fluidique (1) à côté de la soupape de commande (2) de telle sorte qu'elle puisse être commandée depuis le côté extérieur du bloc fluidique (1), la soupape de conduit de travail (3) pouvant être commutée entre une position dans laquelle le conduit de travail (6) est ouvert et une position dans laquelle le conduit de travail (6) est fermé.

11. Bloc fluidique (1) selon l'une quelconque des revendications 1 à 10, dans lequel
la soupape de commande (2) et/ou la soupape de conduit de travail (3) sont une soupape jetable pour usage unique.

12. Bloc fluidique (1) selon l'une quelconque des revendications 1 à 11, comprenant
un conduit pour au moins une ligne électrique pour un instrument électronique à l'extrémité distale du tuyau d'endoscope et
des conduits pour des câbles de traction pour la commande de flexion de l'endoscope.

13. Endoscope comprenant un organe de commande d'endoscope (202) en tant que poignée, la poignée étant formée par le bloc fluidique (1) selon l'une quelconque des revendications 1 à 12, l'un des deux organes conjugués de bloc fluidique (1a) étant intégré dans un organe de poignée.

14. Endoscope comprenant un organe de commande d'endoscope (202) en tant que poignée, la poignée présentant un boîtier dans lequel est disposé le bloc fluidique (1) selon l'une quelconque des revendications 1 à 12.
